# FASCICULE DE BREVET EUROPEEN

(11) **EP 0 426 520 B1**
(45) Date de publication et mention de la délivrance du brevet: **15.06.1994**
(21) Numéro de dépôt: 90402850.3
(22) Date de dépôt: 12.10.1990
(51) Int. Cl.: A61K 7/06, A61K 7/48

(54) **Composition de lavage des matières kératiniques**
Waschmittel für Keratinmaterialien
Cleaning composition of keratinous material

(30) Priorité: 13.10.1989 FR 8913459
(43) Date de publication de la demande: 08.05.1991
(73) Titulaire: L'OREAL, 75008 Paris (FR)
(72) Inventeur: Dubief, Claude, F-78150 Le Chesnay (FR)
(74) Mandataire: Casalonga, Axel

(56) Documents cités:
- EP-A- 0 074 264
- EP-A- 0 331 915
- EP-A- 0 362 048
- GB-A- 2 188 060
- GB-A- 2 211 192
- US-A- 3 659 025

## Description

La présente invention est relative à des compositions de lavage des matières kératiniques, telles que plus particulièrement les cheveux et/ou la peau, et comprenant dans un milieu aqueux, au moins une silicone, un agent tensio-actif et un scléroglucane éventuellement traité au glyoxal, ainsi qu'au procédé de lavage mettant en oeuvre ces compositions.

Les compositions de lavage des matières kératiniques sont bien connues dans l'état de la technique.

On a en particulier déjà proposé d'utiliser de telles compositions contenant en plus de l'agent tensio-actif, des silicones.

On connaît également de telles compositions contenant, à titre d'agents de suspension et épaississants, des acides polyacryliques réticulés tels que, notamment, les produits vendus sous la dénomination de Car- bopol, des celluloses telles que l'hydroxypropylcellulose ou bien de la gomme de xanthane.

Ces agents de mise en suspension et épaississants présentent, cependant, un certain nombre de problèmes, notamment en raison du fait que les compositions les contenant présentent parfois une stabilité insuffisante due, notamment, à une mauvaise compatibilité avec les agents tensio-actifs habituellement utilisés dans les compositions de lavage.

On a également constaté que les propriétés cosmétiques de telles compositions et, notamment, les shampooings, n'étaient pas suffisamment performantes. C'est ainsi, qu'en utilisant, comme agents de suspension et épaississants, des acides polyacryliques réticulés ou des dérivés cellulosiques, on constate souvent une séparation de phases conduisant à des shampooings hétérogènes et instables.

Les compositions contenant de la gomme de xanthane présentent des propriétés détergentes insuffisantes.

On recherche de ce fait des compositions de lavage des matières kératiniques et plus particulièrement de la peau et des cheveux, présentant de bonnes propriétés de stabilité et conférant aux matières traitées des propriétés cosmétiques intéressantes tout en ayant de bonnes propriétés détergentes.

La demanderesse a découvert, ce qui fait l'objet de l'invention, qu'en utilisant dans des compositions de lavage à base de silicones insolubles définies ci-après et d'agents tensio-actifs détergents, un scléroglucane éventuellement traité au glyoxal, les compositions présentaient une très bonne homogénéité, une granulométrie très fine et une bonne stabilité dans le temps.

Par ailleurs, ces compositions confèrent aux cheveux et/ou à la peau une grande douceur et possèdent des propriétés détergentes améliorées.

Ces compositions, en plus de leurs propriétés lavantes, possèdent des propriétés de conditionnement des cheveux, à savoir que les cheveux traités sont brillants, se peignent facilement et sont doux au toucher.

L'invention a donc pour objet de telles compositions de lavage à base de silicones définies ci-après, d'agents tensio-actifs détergents et de scléroglucanes, homogènes et stables dans le temps et présentant de bonnes propriétés de détergence tout en conférant aux matières traitées de la douceur.

Un autre objet de l'invention est constitué par le procédé de lavage mettant en oeuvre de telles compositions.

D'autres objets de l'invention apparaîtront à la lecture de la description et des examples qui suivent.

Les compositions de lavage des matières kératiniques et en particulier des cheveux et de la peau, conformes à l'invention, comprennent dans un milieu aqueux, au moins une silicone, au moins un agent tensio-actif anionique, non-ionique ou amphotère, possédant des propriétés détergentes, au moins un scléroglucane éventuellement traité au glyoxal et au moins une silicone non soluble dans le milieu aqueux, non réactive avec celui-ci, se présentant sous forme d'huiles, de résines ou de gommes, et choisies parmi:
A) les silicones volatiles possédant un point d'ébullition compris entre 60°C et 260°C,
B) des silicones non volatiles choisies en particulier parmi les polyalkylsiloxanes, les polyarylsiloxanes, les polyalkylarylsiloxanes, les copolymères de polyéthersiloxanes organomodifiés ou non, des gommes et résines de silicones, certaines polysiloxanes organomodifiées ainsi que leurs mélanges.

Les silicones volatiles sont plus particulièrement choisies parmi :
a) les silicones cycliques comportant de 3 à 7 atomes de silicium et de préférence 4 à 5 atomes de silicium, telles que plus particulièrement l'octaméthylcyclotétrasiloxane, vendu sous la dénomination VOLATILE SILICONE 7207 par UNION CARBIDE ou SILBIONE 70045 V 2, vendu par la Société RHONE POULENC, ou le décaméthylcyclopentasiloxane, vendu sous la dénomination VOLATILE SILICONE 7158 par la Société UNION CARBIDE, ou bien SILBIONE 70045 V 5, vendu par la Société RHONE POULENC, ainsi que leurs mélanges.

On peut également citer les cyclopolymères tels que le diméthylsiloxane/méthylalkylsiloxane et notamment la silicone volatile FZ 3109, vendue par la Société UNION CARBIDE, présentant la structure : 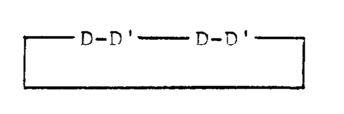 avec 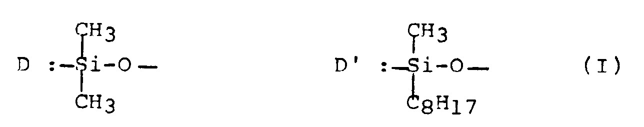
b) les silicones volatiles linéaires, ayant 2 à 9 atomes de silicium et ayant une viscosité inférieure ou égale à 5x10-^{s} m²/s à 25°C. Des silicones de ce type sont en particulier constituées par l'hexaméthyldisiloxane, vendu sous la dénomination SILBIONE 70 041 V 0,65 par la Société RHONE POULENC. Des silicones entrant dans cette classe sont également décrites dans l'article publié dans Cosmetics and toiletries, Vol. 91, Jan. 76, p. 27-32 - TODD & BYERS, "Volatile Silicone fluids for cosmetics".

Les silicones non volatiles mentionnées ci-dessus, sont choisies plus particulièrement parmi les polyalkylsiloxanes, parmi lesquels on peut citer principalement les polydiméthylsiloxanes linéaires à groupement terminaux triméthylsilyle, ayant une viscosité de 5x10⁻⁶ à 2,5 m²/s à 25°C, de préférence 1x10⁻⁵ à 1 m²/s.

Parmi ces polyalkylsiloxanes, on peut citer, à titre non limitatif, les produits commerciaux suivants :
- les huiles SILBIONE des séries 70 047 et47, commercialisées par la Société RHONE POULENC, telles que l'huile 47 V 500.000 de la Société RHONE POULENC;
- les huiles de la série 200 de la Société DOW CORNING;
- les huiles VISCASIL de la Société GENERAL ELECTRIC et certaines huiles des séries SF (SF 96, SF 18) de la Société GENERAL ELECTRIC.

On peut également citer les polydiméthylsiloxanes linéaires à groupements terminaux trihydroxysilyles, tels que plus particulièrement les huiles de la série 48 V de la Société RHONE POULENC.

Dans cette classe de polyalkylsiloxanes, on peut citer les produits vendus sous les dénominations ABIL WAX 9800 et 9801 par la Société GOLDSCHMIDT, qui sont des polyalkyl(C₁-C₂ₒ)siloxanes.

Parmi les polyalkylarylsiloxanes, on peut citer les polyméthylphénylsiloxanes, les polydiméthyldiphényl- siloxanes linéaires et/ou ramifiés, ayant une viscosité de 1x10⁻⁵ à 5x10⁻² m²/s à 25°C.

Parmi ces polyalkylarylsiloxanes, on peut citer, par exemple et sans limitation, les produits commerciaux suivants :
. les huiles SILBIONE de la série 70 641 de la Société RHONE POULENC, telles que les huiles SILBIONE 70 641 V 30 et 70 641 V 200 de la Société RHONE POULENC;
. les huiles RHODORSIL 70 633 V 30 et 763 de la Société RHONE POULENC;
l'huile DC 556 Cosmetic Grade Fluid de la Société DOW CORNING;
. les silicones de la série PK de la Société BAYER, comme le produit PK20;
. les silicones des séries PN, PH de la Société BAYER, comme les produits PN 1000 et PH 1000;
. certaines huiles des séries SF de la Société GENERAL ELECTRIC, telles que les produits SF 1023, SF 1154, SF 1250, SF 1265.

Parmi les copolymères de polyéthersiloxanes modifiés ou non, ou peut citer les copolymères d'oxyde d'éthylène et/ou de propylène avec un diorganosiloxane, tels que plus particulièrement, le produit dénommé diméthicone copolyol, vendu par la Société DOW CORNING sous la dénomination DC 1248 et l'alkyl(C₁₂)méthicone copolyol, vendu par la Société DOW CORNING sous la dénomination Q2 5200; les huiles SILWET L 722, L 7500, L 77, L 711, de la Société UNION CARBIDE.

Les gommes de silicone utilisables, conformément à l'invention, sont des polydiorganosiloxanes ayant des masses moléculaires élevées, comprises entre 200.000 et 1.000.000, utilisés seuls ou en mélange dans un solvant. Ce solvant peut être choisi parmi les silicones volatiles définies ci-dessus, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On peut citer, à titre d'exemple, les produits suivants : les gommes poly(diméthylsiloxane/méthylvinylsiloxane), poly(diméthylsiloxane/diphénylsiloxane), poly(diméthylsiloxane/phénylméthylsiloxane), poly (diméthylsi- loxane/diphénylsiloxane/méthylvinylsiloxane).

Les produits plus particulièrement utilisables sont des mélanges, tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 vendu par la Société DOW CORNING.
. les mélanges formés à partir d'une gomme polydiméthylsiloxane avec une silicone cyclique, tel que le produit SF 1214 Silicone Fluid de la Société GENERAL ELECTRIC (qui est une gomme SE 30, correspondant à une diméthicone, ayant un poids moléculaire de 500.000 solubilisée dans la si licone SF 1202 Silicone Fluid (correspondant au décaméthylcyclopentasiloxane)).
. les mélanges de deux PDMS de viscosités différentes, notamment d'une gomme PDMS et d'une huile PDMS, tels que les produits SF 1236 et CF 1241 de la Société GENERAL ELECTRIC. Le produit SF 1236 est le mélange d'une gomme SE 30 définie ci-dessus d'une viscosité de 20 m²/s et d'une huile SF 96 d'une viscosité de 5.10⁻⁶ m²/s (15% de gomme SE 30 et 85% d'huile SF 96).

Le produit CF 1241 est le mélange d'une gomme SE 30 (33%) et d'une PDMS (67%) de viscosité 10-³ m²/s.

Les résines d'organopolysiloxanes utilisables conformément à l'invention, sont des systèmes siloxaniques réticulés renfermant les unités R₂SiO_{2/2}, RSiO_{3/2} et SiO_{4/2}, dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination DOW CORNING 593 ou ceux vendus sous les dénominations SILICONE FLUID SS 4230 et SS 4267 par la Société GENERAL ELECTRIC et qui sont des "diméthyl/triméthylpolysiloxane".

Les silicones organomodifiées sont des silicones définies ci-dessus et comportant dans leur structure un ou plusieurs groupements organofonctionnels directement fixés sur la chaîne siloxanique ou fixés par l'intermédiaire d'un radical hydrocarboné, ces groupements étant choisis parmi :

1) des groupements thiols, comme les produits GP 72 A et GP 71 de la Société GENESEE,
2) des groupements carboxylates, comme c'est le cas des produits décrits dans le brevet EP-A-186 507 de la Société CHISSO CORPORATION,
3) des groupements alcoxylés, comme le produit vendu sous la dénomination Silicone copolymer F-755 de la Société SWS SILICONES, et ABIL WAX 2428, 2434 et 2440 de la Société GOLDSCHMIDT,
4) des groupements hydroxylés, comme les polyorganosiloxanes à fonction hydroxyalkyle, décrits dans la demande de brevet français n° 85 163 34, et répondant à la formule : 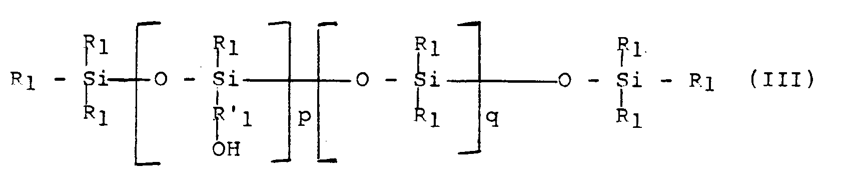 dans laquelle :
   les radicaux R₁, identiques ou différents, sont choisis parmi les radicaux méthyle et phényle, au moins 60% en mole des radicaux R₁ désignant méthyle;
   le radical R'₁ est un chaînon alkylène divalent hydrocarboné en C₂-C₁₈;
   p est compris entre 1 et 30 inclus;
   q est compris entre 1 et 150 inclus;
5) des groupements acyloxyalkyle, comme par exemple les polyorganosi loxanes décrits dans la demande de brevet FR-A 88 17 433, répondant à la formule : 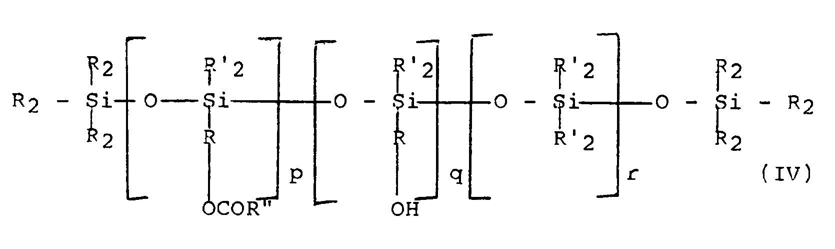 dans laquelle :
   R₂ désigne un groupement méthyle, phényle, -OCOR", hydroxyle, un seul des radicaux R₂ par ato-me de silicium peut être OH;
R'₂ désigne méthyle, phényle, au moins 60% en mole de l'ensemble des radicaux R₂ et R'₂ désignant méthyle;
R" désigne un alkyle ou alkényle en C₈-C₂₀;
R désigne un radical alkylène hydrocarboné divalent, linéaire ou ramifié, en C₂-C₁₈;
r est compris entre 1 et 120 inclus;
p est compris entre 1 et 30;
q est égal à 0 ou est inférieur à 0,5p, p+q étant compris entre 1 et 30; les polyorganosiloxanes de formule (IV) pouvant contenir des groupements 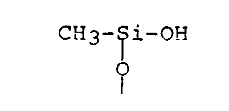 dans des proportions ne dépassant pas 15% de la somme p+q+r.

Les composés de formule (IV) peuvent être préparés par estérification de polyorganosiloxanes à fonction hydroxyalkyle de formule (III) ci-dessus.

L'estérification s'effectue de façon connue, avec un acide R"COOH ou l'anhydride d'acide, à une température comprise entre 100 et 250°C en présence éventuellement d'un catalyseur comme le chlorure d'aluminium ou le chlorure de zinc ou d'un acide fort comme l'acide chlorhydrique ou l'acide sulfurique.

On peut également effectuer une transestérification par chauffage à100-150°Cd'unester méthylique de formule R"COOCH₃ et d'un diorganopolysiloxane de formule (III), en présence d'un catalyseur acide comme l'acide paratoluènesulfonique ou une terre acide de type Montmorillonite (KATALYSATOR KSF/O, vendu par SUDCHEMIE - A.G. MUNCHEN).

6) des groupements anioniques de type carboxylique tels que les groupements alkylcarboxyliques comme ceux présents dans le produit X-22-3701 E de la Société SHIN-ETSU; 2-hydroxyalkylsulfonate; 2-hy- droxyalkylthiosulfate tels que les produits vendus par la Société GOLDSCHMIDT sous les dénominations "ABIL S201 " et "ABIL S 255".

Les polyorganosiloxanes plus particulièrement préférés, conformément à l'invention, sont des silicones non volatiles choisies dans la famille des polyalkylsiloxanes linéaires à groupements terminaux triméthylsi- lylés telles que les produits vendus sous la dénomination SILBIONE 70047 et 47 V 500.000 par la Société RHONE POULENC ou les polyalkylarylsiloxanes telles que l'huile SILBIONE 70641 V 200 commercialisée par la Société RHONE POULENC;
. des mélanges d'organosiloxanes et de silicones cycliques tels que le produit Q2 1401 vendu par la Société DOW CORNING et le produit SF 1214 vendu par la Société GENERAL ELECTRIC;
. la résine d'organopolysiloxanes vendue sous la dénomination DOW CORNING 593.

Le mélange de deux PDMS de viscosités différentes, tels que les produits vendus sous la dénomination CF 1241 par la Société GENERAL ELECTRIC, est utilisé de façon particulièrement préférée.

Les scléroglucanes utilisés conformément à l'invention, sont des polysaccharides neutres d'origine microbienne, obtenus par fermentation aérobie d'un milieu glucosé par un champignon du type Sclerotium et présentant la structure d'un homopolymère de D-glucopyranose.

Les scléroglucanes répondent à la formule : 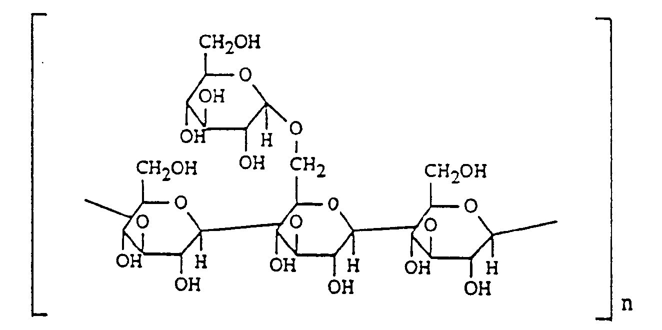 où n (degré de polymérisation) varie de 500 à 1600.

Les scléroglucanes plus particulièrement utilisés, conformément à l'invention, sont représentés par les produits vendus sous la dénomination ACTIGUM CS par la Société SANOFI BIO INDUSTRIES et en particulier ACTIGUM CS 11 et sous la dénomination AMIGEL par la Société ALBAN MULLER INTERNATIONAL.

D'autres scléroglucanes tels que celui traité au glyoxal décrit dans la demande de brevet français n° 2.633.940, peuvent également être utilisés.

Les agents tensio-actifs utilisés dans les compositions conformes à l'invention, sont choisis parmi les agents tensio-actifs anioniques, amphotères, zwittérioniques, non ioniques ou leurs mélanges.

Parmi les agents tensio-actifs anioniques, on peut citer plus particulièrement les sels alcalins, les sels d'ammonium, les sels d'amine ou les sels d'aminoalcools des composés suivants :
- les alkylsulfates, alkyléthersulfates, alkylamide éthersulfates, alcanolamidesulfates, alkylarylpolyéther- sulfates, monoglycérides sulfates;
- les alkylsulfonates, alkylarylsulfonates, alkylamidesulfonates, oléfines sulfonates, paraffines sulfonates;
- les alkylsulfosuccinates, alkyléthersulfosuccinates, alkylamides sulfosuccinates;
- les alkylsulfosuccinamates;
- les alkylsulfoacétates;
- les alkylphosphates, alkylétherphosphates;
- les acylsarcosinates, les acylpolypeptidates, les acyliséthionates, les N-acyltaurates.

Le radical alkyle ou acyle de ces différents composés est généralement constitué par une chaîne carbonée, comportant 12 à 20 atomes de carbone.

Parmi les agents tensio-actifs anioniques, on peut également citer les sels d'acides gras, tels que les acides oléique, ricinoléique, palmitique, stéarique; les acides d'huile de coprah ou d'huile de coprah hydrogénée; les acyl lactylates, dont le radical acyle comporte 8 à 20 atomes de carbone.

Parmi les agents tensio-actifs non ioniques, on peut citer les alcools, alkylphénols et acides gras polyoxyéthylénés, polypropoxylés ou polyglycérolés à chaîne grasse, comportant 8 à 18 atomes de carbone. Le nombre de groupements oxyde d'éthylène, oxyde de propylène, étant compris entre 2 et 50 et le nombre de groupement glycérol étant compris entre 2 et 30.

On peut également citer les copolymères d'oxydes d'éthylène et de propylène, des condensats d'oxydes d'éthylène et de propylène sur des alcools gras, des amides gras polyéthoxylés (de préférence à 2 à 30 moles d'oxyde d'éthylène), des amines grasses polyéthoxylées (de préférence à 2 à 30 moles d'oxyde d'éthylène), des éthanolamides, des esters d'acides gras de glycol, des esters d'acides gras du sorbitan oxyéthylénés (de préférence à 2 à 30 moles d'oxyde d'éthylène) ou non, des esters d'acides gras du saccharose, des esters d'acides gras de polyéthylène glycol, des triesters phosphoriques, des esters d'acides gras de dérivés du glucose, des oxydes d'amines tels que les oxydes d'alkylamines ou de N-acylamidopropylmorpholine.

Les alcools gras polyoxyéthylénés ou polyglycérolés préférés sont l'alcool oléique oxyéthyléné à 10 moles d'oxyde d'éthylène, l'alcool laurique oxyéthyléné à 12 moles d'oxyde d'éthylène, le nonylphénol oxyéthyléné à 9 moles d'oxyde d'éthylène, l'alcool oléique polyglycérolé à 4 moles de glycérol; l'ester d'acides gras du sorbitan oxyéthyléné préféré est le monolaurate de sorbitan polyoxyéthyléné à 20 moles d'oxyde d'éthylène.

D'autres composés entrant dans cette classe sont des composés répondant aux formules (VI) à (VIII) ci-après et/ou préparés selon les procédés décrits ci-après.
a) 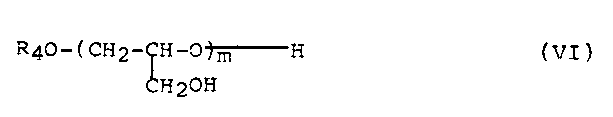 dans laquelle R₄ désigne un radical ou un mélange de radicaux alkyle contenant 10 à 14 atomes de carbone et m est un nombre entier ou décimal de 2 à 10 et de préférence de 3 à 6. Ces composés peuvent être préparés selon le procédé décrit dans le brevet FR-A-1 477 048;
b) 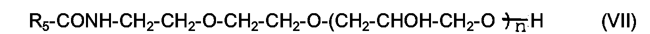 dans laquelle R₅ désigne un radical ou un mélange de radicaux alkyle et/ou alkényle ayant de 11 à 17 atomes de carbone et n désigne un nombre entier ou décimal de 1 à 5 et de préférence de 1,5 à 4. Ces composés de formule (VII) peuvent être préparés selon le procédé décrit dans le brevet FR-A-2 328 763;
c) 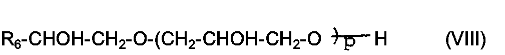 dans laquelle R₆ désigne un radical aliphatique, cycloaliphatique, arylaliphatique, ayant de préférence 7 à 21 atomes de carbone, et leurs mélanges, les chaînes aliphatiques désignant en particulier des chaînes alkyle pouvant comporter 1 à 6 groupements éther, thioéther et/ou hydroxyméthylène et p est compris entre 1 et 10 inclus.

Ces composés sont préparés par condensation en catalyse alcaline, de 2 à 10 et de préférence de 2,5 à 6 moles de glycidol sur un alpha-diol ou un mélange d'alpha-diols en C₁₀-C₁₄, à la température de 120 à 180°C et de préférence de 140 à 160°C, le glycidol étant ajouté lentement, selon le procédé décrit dans le brevet FR-A-2 091 516;
d) les composés préparés par condensation, en catalyse acide, de 2 à 10 et de préférence de 2,5 à 6 moles de glycidol par mole d'alcool ou d'alpha-diol contenant 10 à 14 atomes de carbone à une température de 50 à 120°C, le glycidol étant ajouté lentement à l'alcool ou à l'alpha-diol. Le procédé de préparation de ces composés est décrit plus particulièrement dans le brevet FR-A-2 169 787;
e) les composés de poly(hydroxypropyléther) préparés par polyaddition de monochlorhydrine du glycérol sur un composé organique polyhydroxylé, en présence d'une base forte, avec élimination au fur et à mesure de l'eau par distillation, décrits plus particulièrement dans le brevet FR-A-2 574 786.

Parmi les agents tensio-actifs non ioniques de la famille des polyhydroxypropyléthers décrits dans les paragraphes (a) à (e) ci-dessus, les composés préférés sont représentés par les formules : 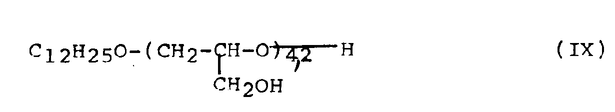 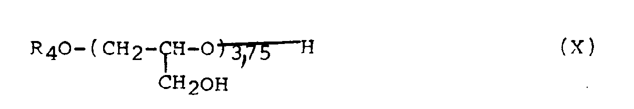 dans laquelle R₄ désigne un mélange de radicaux alkyle en C₁₀H₂₁ et C₁₂H₂₅;
. les composés préparés par condensation en catalyse alcaline, de 3,5 moles de glycidol sur un alpha-diol ayant 12 atomes de carbone, selon le procédé décrit dans le brevet FR-A-2 091 516;
. les composés répondant à la formule : 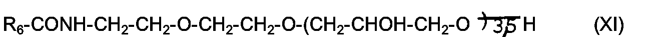 dans laquelle R₆ désigne un mélange de radicaux comprenant les radicaux alkyle et alcényle suivants : CₗₗH₂₃, C₁₃H₂₇, les radicaux dérivés des acides gras du coprah et le radical dérivé de l'acide oléique.

Les composés préparés par condensation de 3,5 moles de glycidol sur un mélange d'alpha-diols en Cₗₗ-C₁₄, décrits plus particulièrement dans le brevet FR-A-2 091 516 et le poly(hydroxypropyléther) obtenu par condensation de la monochlorhydrine du glycérol (2,5 moles) en présence de soude sur le dodécanediol-1,2, sont plus particulièrement préférés.

Les agents tensio-actifs amphotères et zwittérioniques sont choisis parmi les dérivés d'amines secondaires ou tertiaires aliphatiques, dans lesquels le radical aliphatique est une chaîne linéaire ou ramifiée comportant de 8 à 18 atomes de carbone et contient au moins un groupement anionique hydrosolubilisant carboxylique, sulfonate, sulfate, phosphate ou phosphonate et les alkylbétaïnes, les sulfobétaïnes, les amidobétaïnes ou les amidosulfobétaïnes.

Parmi ces composés, on peut citer les produits vendus sous la dénomination de MIRANOL, décrits plus particulièrement dans les brevets US-A-2 528 378 et 2 781 354 et classés dans le dictionnaire CTFA, 3ème édition, 1982, sous la dénomination de Amphocarboxyglycinates et Amphocarboxypropionates.

Ces produits présentent les structures suivantes :
- les amphocarboxyglycinates 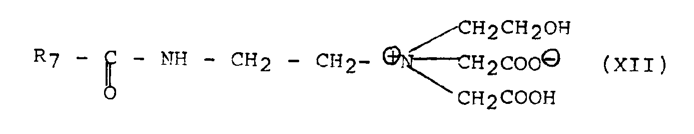 dans laquelle R₇ désigne un radical alkyle dérivé du coprah, un radical heptyle, nonyle ou undécyle;
- les amphocarboxypropionates 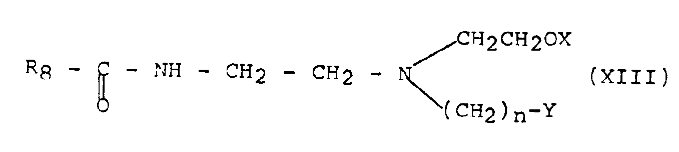 dans laquelle :
   n désigne 1 ou 2 ;
   X désigne le groupement -CH₂CH₂COOH ou hydrogène;
   Y désigne -COOH ou le radical 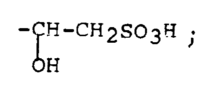
   Rₐ désigne le radical alkyle dérivé du coprah, un radical alkyle en C₇, Cg, C₁₁, C₁₃, un radical alkyle en C₁₇ et sa forme iso, un radical en C₁₇ insaturé, un radical alkyle dérivé de l'huile de lin.

Les alkylbétaïnes sont choisies de préférence parmi les alkyl(C₁₀-C₂₀)bétaïnes.

Conformément à l'invention, on utilise de préférence des mélanges d'agents tensio-actifs et en particulier des mélanges d'agents tensio-actifs anioniques et d'agents tensio-actifs amphotères, zwittérioniques ou non-ioniques. Un mélange particulièrement préféré est un mélange constitué d'un agent tensio-actif anionique et d'un agent tensio-actif zwittérionique.

On utilise de préférence un agent tensio-actif anionique choisi parmi les alkyl(C₁₂-C₁₄)sulfatesde sodium, de triéthanolamine ou d'ammonium, les alkyl (C₁₂-C₁₄)éthersulfates de sodium oxyéthylénés à 2,2 moles d'oxyde d'éthylène, le cocoyliséthionate de sodium et l'a-oléfine(C₁₄-C₁₆)sulfonate de sodium, ainsi que leurs mélanges, avec
- soit un agent tensio-actif amphotère tel qu'un amphocarboxyglycinate défini par la formule (XII), dans laquelle R₇ désigne un radical alkyle dérivé du coprah, encore dénommé cocoamphocarboxyglycinate et vendu par la Société MIRANOL sous la dénomination de MIRANOL C2M CONC en solution aqueuse à 38% de matière active;
- soit un agent tensio-actif zwittérionique tel que la laurylbétaïne vendue par la Société HENKEL sous la dénomination DEHYTON AB 30 en solution aqueuse à 32% de matière active.

Les silicones sont utilisées dans les compositions conformes à l'invention, dans des proportions comprises entre 0,2 et 20% en poids par rapport au poids total de la composition et de préférence entre 0,2 et 10% en poids.

Les scléroglucanes sont utilisés dans des proportions suffisantes pour assurer l'homogénéité et l'épaississement des compositions de l'invention, et de préférence dans des proportions comprises entre 0,1 et 5% en poids et en particulier entre 0,2 et 3% en poids par rapport au poids total de la composition.

Les agents tensio-actifs sont utilisés dans les compositions conformes à l'invention dans des proportions suffisantes pour conférer un caractère détergent à la composition et de préférence comprises entre 5 et 50% en poids par rapport au poids total de la composition et en particulier entre 8 et 30% en poids.

Lorsque les agents tensio-actifs amphotères ou zwittérioniques sont utilisés en mélange avec les agents tensio-actifs anioniques, ils représentent jusqu'à 50%, et de préférence 5 à 30% en poids du poids total de la quantité d'agents tensio-actifs présents dans la composition.

Lorsque les agents tensio-actifs non ioniques sont utilisés en mélange avec les agents tensio-actifs anioniques, ils représentent jusqu'à 90% et de préférence de 5 à 50% en poids du poids total de la quantité d'agents tensio-actifs présents dans la composition.

Le pH des compositions conformes à l'invention est généralement compris entre 3 et 9 et de préférence entre 4 et 8.

Le milieu aqueux peut être constitué uniquement par de l'eau ou par un mélange d'eau et d'un solvant cosmétiquement acceptable, tel que des alcools inférieurs en Cq-C₄, comme l'éthanol, l'isopropanol, le n-bu- tanol; les alkylèneglycols, comme l'éthylèneglycol, les éthers de glycols.

Les compositions conformes à l'invention peuvent contenir en plus de l'association définie ci-dessus, des agents régulateurs de viscosité tels que des épaississants, des électrolytes, des hydrotropes, parmi lesquels on peut citer le chlorure de sodium, le xylènesulfonate de sodium.

Les agents régulateurs de viscosité sont utilisés dans les compositions conformes à l'invention, dans des proportions allant jusqu'à 6% en poids par rapport au poids total de la composition.

Les compositions conformes à l'invention peuvent éventuellement contenir, en outre, d'autres agents ayant pour effet d'améliorer les propriétés cosmétiques des cheveux et de la peau, à condition de ne pas altérer la stabilité des compositions. On peut utiliser, parexemple, les agents tensio-actifs cationiques, les polymères anioniques ou cationiques ou amphotères ou des protéines éventuellement quaternisées.

Les compositions peuvent également contenir différents adjuvants habituellement utilisés en cosmétique, tels que des parfums, des conservateurs, des séquestrants, des stabilisateurs de mousse et des agents acidifiants ou alcalinisants bien connus en cosmétique.

Les compositions conformes à l'invention sont plus particulièrement utilisées comme shampooings pour le lavage et le conditionnement des cheveux et ils sont appliqués, dans ce cas-là, sur des cheveux humides dans des quantités efficaces pour les laver, cette application étant suivie d'un rinçage.

Les compositions conformes à l'invention sont également utilisables comme gels douche pour le lavage des cheveux et de la peau, auquel cas ils sont appliqués sur la peau et les cheveux humides et sont rincés après application.

Les exemples suivants sont destinés à illustrer l'invention.

Les marques déposées sont reconnues en tant que telles.

### EXEMPLE 1

On prépare un shampooing de composition suivante :
- Scléroglucane vendu sous la dénomination ACTIGUM CS 11 par la Société SANOFI BIO INDUSTRIES à 90% de MA 0,9 g MA
- Xylènesulfonate de sodium vendu sous la dénomination ELTESOL SX 93 par la Société MARCHON à 93% de MA 1,86 g MA
- Mélange de deux PDMS de viscosités différentes, vendu sous la dénomination CF 1241 par la Société GENERAL ELECTRIC 3,0 g MA
- Laurylbétaïne vendue sous la dénomination DEHYTON AB 30 par la Société HENKEL en solution aqueuse à 32% de MA 2,0 g MA
- Alkyl(C₁₂-C₁₄)/70-30)sulfate de triéthanolamine en solution aqueuse à 40% de MA 10,0 g MA
- Conservateur qs
- Chlorure de sodium 4,0 g
- Eau qsp 100,0 g

Les cheveux lavés avec ce shampooing se peignent facilement, sont brillants et présentent un toucher doux.

### EXEMPLE 2

On prépare un shampooing de composition suivante :
- Scléroglucane vendu sous la dénomination ACTIGUM CS 11 par la Société SANOFI BIO INDUSTRIES à 90% de MA 0,9 g MA
- Laurylsulfate d'ammonium 8,0 g MA
- Silicone DC 200-350 CSt vendue par DOW CORNING 3,0 g
- HCI qs pH = 5
- Eau qsp 100,0 g

### EXEMPLE 3

On prépare un shampooing de composition suivante :
- Sléroglucane vendu sous la dénomination ACTIGUM CS 11 par la Société SANOFI BIO INDUSTRIES à 90% de MA 1,35 g MA
- Mélange d'un diméthiconol et d'une cyclométhicone vendu sous la dénomination Q2 1401 par la Société
DOW CORNING 5,0 g MA
- Alkyl(C₁₂-C₁₄/70-30)sulfate de triéthanolamine en solution aqueuse à 40% de MA 2,0 g MA
- Alkyl(C₁₂-C₁₄)éthersulfate de sodium oxyéthyléné à 2,2 moles d'oxyde d'éthylène vendu à 25% de MA 10,0 g MA
- Conservateur, parfum, qs
- HCI qs pH = 6,4
- Eau qsp 100,0 g

### EXEMPLE 4

On prépare un shampooing de composition suivante :
- Scléroglucane vendu sous la dénomination ACTIGUM CS 11 par la Société SANOFI BIO INDUSTRIES à 90% de MA 0,9 g MA
- Silicone DC 593 vendue par la Société DOW CORNING 0,5 g
- Laurylbétaïne vendue sous la dénomination DEHYTON AB 30 par la Société HENKEL en solution aqueuse à 32% de MA 2,0 g MA
- Alkyl(C₁₂-C₁₄/70-30)sulfate de triéthanolamine en solution aqueuse à 40% de MA 10,0 g MA
- Conservateur, parfum, qs
- Triéthanolamine qs pH = 6,8
- Eau qsp 100,0 g

Les cheveux lavés avec ce shampooing se peignent facilement, sont brillants et présentent un toucher doux.

### EXEMPLE 5

On prépare un shampooing de composition suivante :
- Scléroglucane vendu sous la dénomination ACTIGUM CS 11 par la Société SANOFI BIO INDUSTRIES à 90% de MA 2,25 g MA
- Tensio-actif non ionique poly(hydroxypropyléther) préparé par condensation en catalyse alcaline de 3,5 moles de glycidol sur un alpha-diol ayant 12 atomes de carbone, selon le procédé décrit dans le brevet FR-A-2 091 516 5,0 g MA
- 0léfine(C₁₄-C₁₆)sulfonatede sodium 5,0 g MA
- Huile SILBIONE 70641 V 200 vendue par la Société RHONE POULENC 3,5 g
- Conservateur, parfum, qs
- HCI qs pH = 7
- Eau qsp 100,0 g

### EXEMPLE 6

On prépare un gel douche de composition suivante :
- Scléroglucane vendu sous la dénomination AMIGEL par la Société ALBAN MULLER INTERNATIONAL 1,5 g MA
- Alkyl(C₁₂-C₁₄)éthersulfate de sodium oxyéthyléné à 2,2 moles d'oxyde d'éthylène, vendu à 25% de MA 8,0 g MA
- Cocoamphocarboxyglycinate vendu sous la dénomination MIRANOL C2M CONC par la Société MIRANOL, en solution aqueuse à 38% de MA 5,0 g MA
- Huile RHODORSIL 763 vendue par la Société RHONE POULENC 0,5 g
- Conservateur, parfum, qs
- HCI qs pH = 8
- Eau qsp 100,0 g

### EXEMPLE 7

On prépare un gel douche de composition suivante :
- Scléroglucane vendu sous la dénomination ACTIGUM CS 11 par la Société SANOFI BIO INDUSTRIES à 90% de MA 2,7 g MA
- Alkyl(C₁₂-C₁₄/70-30)sulfatede triéthanolamine en solution aqueuse à 40% de MA 8,0 g MA
- Tensio-actif non ionique poly(hydroxypropyléther) préparé par condensation en catalyse alcaline de 3,5 moles de glycidol sur un alpha-diol ayant 12 atomes de carbone, selon le procédé décrit dans le brevet FR-A-2 091 516 5,0 g MA
- Mélange (84/8 poids) de cocoyl iséthionate de sodium/iséthionate de sodium, vendu sous la dénomination ARLATONE SCI par la Société ICI 2,0 g
- Oxyde d'amine tertiaire : oxyde de di(hydoxyéthyl)alkyl (coprah) amine, vendu sous la dénomination AROMOX C12 par la Société ARMAK à 50% de MA 1,0 g MA
- polysiloxane à fonction hydroxyalkyle, de formule :

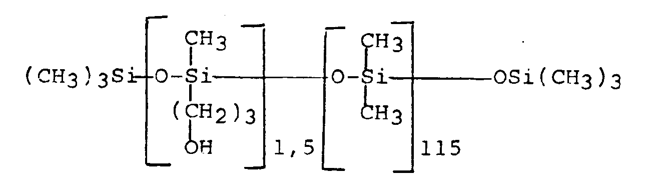

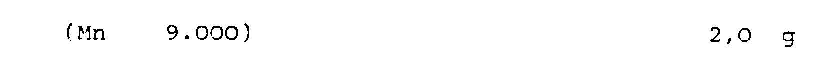

- Conservateur, parfum, qs
- HCI = qs pH = 7
- Eau qsp 100,0 g

### EXEMPLE 8

On prépare un gel douche de composition suivante :
- Lauryléther sulfate de sodium oxyéthyléné à 2,2 moles d'oxyde d'éthylène, vendu à 25% de MA 15,0 g MA
- Polydiméthylsiloxane vendu sous la dénomination HUILE SILBIONE 70047 V 300 par la Société RHONE POULENC 10,0 g
- Cocoamidopropyl bétaïne en solution aqueuse à 35% de MA, vendue sous la dénomination TEGOBE-TAINE HS par la Société GOLDSCHMIDT 8,0 g MA
- Scléroglucane vendu sous la dénomination ACTIGUM CS 11 par la Société SANOFI BIO INDUSTRIES à 90% de MA 0,27 g MA
- Parfum, conservateurs qs
- HCI qs pH = 6,5
- Eau 100,0 g

### EXEMPLE 9

On prépare un shampooing de composition suivante :
- Lauryléther sulfate de sodium oxyéthyléné à 2,2 moles d'oxyde d'éthylène, vendu à 25% de MA 13,0 g MA
- Polydiméthylsiloxane vendu sous la dénomination HUILE SILBIONE 47 V 500.000 par la Société RHONE POULENC 3,0 g
- Cocoamidopropyl bétaïne en solution aqueuse à 35% de MA, vendu sous la dénomination TEGOBE-TAINE HS par la Société GOLDSCHMIDT 10,0 g MA
- Scléroglucane vendu sous la dénomination ACTIGUM CS 11 par la Société SANOFI BIO INDUSTRIES à 90% de MA 0,27 g MA
- Parfum, conservateurs, colorant qs
- Triéthanolamine qs pH = 7
- Eau 100,0 g

## Revendications

1. Composition de lavage des matières kératiniques, en particulier des cheveux et/ou de la peau, caractérisée par le fait qu'elle contient dans un milieu aqueux, au moins :

- un agent tensio-actif anionique, non-ionique ou amphotère, détergent;
- un scléroglucane éventuellement traité au glyoxal, et
- une silicone insoluble dans le milieu aqueux, choisie parmi :
A) les silicones volatiles ayant un point d'ébullition compris entre 60 et 260°C;
B) les silicones non volatiles, choisies parmi :
a) les polyalkylsiloxanes choisis parmi les polydiméthylsiloxanes linéaires à groupements terminaux triméthylsilyle, ayant une viscosité de 5x10-^{s} à 2,5 m²/s à 25°C, et les polydiméthylsiloxanes linéaires à groupements terminaux trihydroxysilyles et les polyalkyl(C₁-C₂ₒ)siloxanes;
b) les polyalkylarylsiloxanes choisis parmi les polyméthylphénylsiloxanes ou les polydiméthyldi- phénylsiloxanes linéaires et/ou ramifiés, ayant une viscosité de 10⁻⁵ à 5x10-² m²/s à 25°C;
c) les copolymères de polyéthersiloxanes modifiés ou non;
d) les gommes de silicones constituées de polydiorganosiloxanes à poids moléculaire élevé compris entre 200.000 et 1.000.000, utilisés seuls ou en mélange dans un solvant choisi parmi les silicones volatiles, les huiles polydiméthylsiloxanes (PDMS), les huiles polyphénylméthylsiloxanes (PPMS), les isoparaffines, le chlorure de méthylène, le pentane, le dodécane, le tridécane, le tétradécane, ou leurs mélanges;
e) les résines d'organopolysiloxanes qui sont des systèmes siloxaniques réticulés, renfermant des unités R₂SiO_{2/2}, RSi0_{3/2} et SiO_{4/2}, dans lesquelles R repésente un groupement hydrocarboné possédant 1 à 6 atomes de carbone ou un groupement phényle;
f) les silicones organomodifiées définies ci-dessus (a à d), comportant sur leur structure générale un ou plusieurs groupements organofonctionnels directement fixés sur la chaîne siloxanique ou fixés par l'intermédiaire d'un radical hydrocarboné et choisis parmi :
(i) des groupements thiols;
(ii) des groupements carboxylates;
(iii) des groupements alcoxylés;
(iv) des groupements hydroxylés;
(v) des groupements acyloxyalkyle;
(vi) des groupements anioniques de type alkylcarboxylique 2-hydroxyalkylsulfonate ou 2-hydroxyalkyl-thiosulfate, cette composition ne contenant pas de polysiloxani à fonction β-cétoes- ter.

2. Composition selon la revendication 1, caractérisée par le fait que la silicone volatile est choisie parmi les silicones cycliques comportant 3 à 7 atomes de silicium et de préférence 4 à 5 atomes de silicium, les cyclopolymères diméthylsiloxane/méthylalkylsiloxane, répondant à la formule : 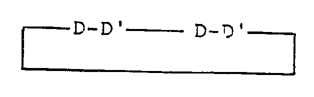 avec 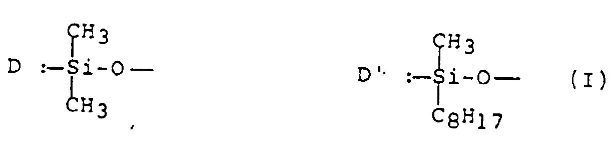 les silicones volatiles linéaires, ayant 2 à 9 atomes de silicium et une viscosité inférieure ou égale à 5x10-^{s} m²/s à 25°C.

3. Composition selon la revendication 2, caractérisée par le fait que les silicones volatiles sont choisies parmi l'octaméthylcyclotétrasiloxane, le décaméthylcyclopantasiloxane.

4. Composition selon la revendication 1, caractérisée par le fait que les gommes de silicone sont choisies parmi les gommes de poly(diméthylsiloxane/méthylvinylsiloxane);de poly(diméthylsiloxane/diphénylsiloxane); de poly(diméthylsiloxane/phénylméthylsiloxane); de poly(diméthylsiloxane/diphénylsiloxane/ méthylvi- nylsiloxane).

5. Composition selon la revendication 1, caractérisée par le fait que les gommes de silicone en mélange dans un solvant, sont choisies parmi les mélanges d'un polydiméthylsiloxane hydroxylé en bout de chaîne et d'un polydiméthylsiloxane cyclique; d'un mélange d'une gomme de polydiméthylsiloxane avec une silicone cyclique; d'un mélange d'une gomme de polydiméthylsiloxane et d'une huile PDMS de viscosités différentes.

6. Composition selon la revendication 1, caractérisée par le fait que les polyorganosiloxanes à groupement hydroxylé sont des polyorganosiloxanes à fonction hydroxyalkyle, répondant à la formule : 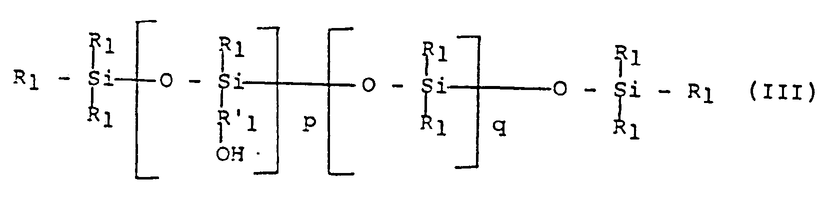 dans laquelle :
les radicaux R_{1'} identiques ou différents, sont choisis parmi les radicaux méthyle et phényle, au moins 60% en mole des radicaux R₁ désignant méthyle;
le radical R'ᵢ est un chaînon alkylène divalent hydrocarboné en C₂-C₁₈;
p est compris entre 1 et 30 inclus;
q est compris entre 1 et 150 inclus.

7. Composition selon l'une quelconque des revendications 1 à 6, caractérisée par le fait que les scléroglucanes sont des polysaccharides neutres d'origine microbienne, obtenus par fermentation aérobie d'un milieu glucosé par un champignon du type Sclérotium, et présentant la structure d'un homopolymère de D-glucopyranose.

8. Composition selon la revendication 7, caractérisée par le fait que les agents tensio-actifs anioniques sont choisis parmi les sels alcalins, les sels d'ammonium, les sels d'amines ou les sels d'aminoalcools des composés suivants :
- les alkylsulfates, alkyléthersulfates, alkylamides éthersulfates, alcanolamidesulfates, alkylarylpolyé- thersulfates, monoglycérides sulfatés;
- les alkylsulfonates, alkylarylsulfonates, alkylamidesulfonates, oléfines sulfonates, paraffines sulfonates;
- les alkylsulfosuccinates, alkyléthersulfosuccinates, alkylamides sulfosuccinates;
- les alkylsulfosuccinamates;
- les alkylsulfoacétates;
- les alkylphosphates, alkylétherphosphates;
- les acylsarcosinates, les acylpolypep- tidates, les acyliséthionates, les N-acyltaurates,
dans lesquels le radical alkyle ou acyle est une chaîne linéaire hydrocarbonée de 12 à 18 atomes de carbone.

9. Composition selon l'une quelconque des revendications 1 à 8, caractérisée par le fait que les agents tensio-actifs anioniques sont choisis parmi les sels des acides oléique, ricinoléique, palmitique, stéarique; des acides d'huile de coprah d'huile de coprah hydrogénée; les acyl lactylates, dans lesquels le radical acyle comporte 8 à 20 atomes de carbone.

10. Composition selon l'une quelconque des revendications 1 à 7, caractérisée par le fait que les agents tensio-actifs non ioniques sont choisis parmi les alcools, alkylphénols et acides gras polyoxyéthylénés, polypropoxylés ou polyglycérolés à chaîne grasse linéaire, comportant 8 à 18 atomes de carbone; les copolymères d'oxydés d'éthylène et de propylène, les condensats d'oxydes d'éthylène et de propylène sur des alcools gras, des amide gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylène glycol, des triesters phosphoriques, des esters d'acides gras de dérivés du glucose, des oxydes d'amines.

11. Composition selon l'une quelconque des revendications 1 à 7, caractérisée par le fait que les agents tensio-actifs non ioniques sont choisis parmi les composés répondant aux formules ou préparés selon les procédés suivants :
a) 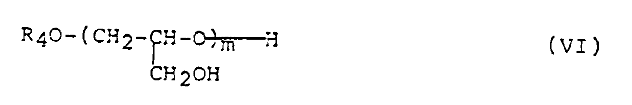 dans laquelle R₄ désigne radical ou un mélange de radicaux alkyle comportant 10 à 14 atomes de carbone et m est un nombre entier ou décimal de 2 à 10 et de préférence de 3 à 6;
b) 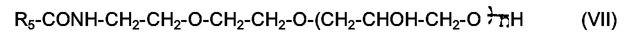 dans laquelle R₅ désigne un radical ou un mélange de radicaux alkyle et/ou alcényle ayant de 11 à 17 atomes de carbone et n désigne un nombre entier ou décimal de 1 à 5 et de préférence de 1,5 à 4;
c) 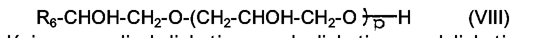 aans laquelle R₆aesigne un raaicai allphatique, cycloallphatique, arylallphatique, ayant ae preference 7 à 21 atomes de carbone, et leurs mélanges, les chaînes aliphatiques désignant des chaînes alkyle pouvant comporter 1 à 6 groupements éther, thioéther et/ou hydroxyméthylène et p est compris entre 1 et 10 inclus;
d) les composés préparés par condensation, en catalyse acide, de 2 à 10 et de préférence de 2,5 à 6 moles de glycidol par mole d'alcool ou d'alpha-diol contenant 10 à 14 atomes de carbone;
e) les composés de poly(hydroxypropyléther) préparés par polyaddition de monochlorhydrine du glycérol sur un composé organique polyhydroxylé, en présence d'une base forte, par élimination au fur et à mesure de l'eau par distillation.

12. Composition selon la revendication 11, caractérisée par le fait que les agents tensio-actifs non ioniques sont choisis parmi les composés répondant aux formules :

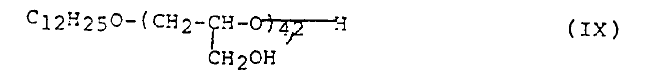

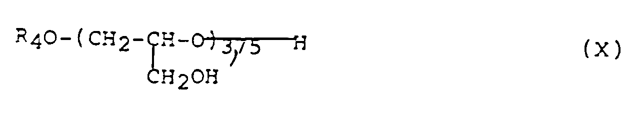

dans laquelle R₄ désigne un mélange de radicaux alkyle en C₁₀H₂₁ et C₁₂H₂₅;
- les composés préparés par condensation en catalyse alcaline, de 3,5 moles de glycidol sur un alpha-diol ayant 12 atomes de carbone;
- les composés répondant à la formule : 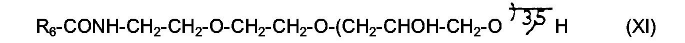 dans laquelle R₆ désigne un mélange de radicaux comprenant les radicaux alkyle et alkényle suivants : C₁₁ H₂₃, C₁₃H₂₇, les radicaux dérivés des acides gras du coprah et le radical dérivé de l'acide oléique;
- les composés préparés par condensation de 3,5 moles de glycidol sur un mélange d'alpha-diols en C₁₁-C₁₄;
- le poly(hydroxypropyléther) obtenu par condensation de la monochlorhydrine du glycérol (2,5 moles) en présence de soude sur le dodécanediol-1,2.

13. Composition selon l'une quelconque des revendications 1 à 7, caractérisée par le fait que les agents tensio-actifs amphotères et zwittérioniques sont choisis parmi les dérivés d'amines secondaires ou tertiaires aliphatiques, dans lesquels le radical aliphatique est une chaîne linéaire ou ramifiée, comportant de 8 à 18 atomes de carbone et contient au moins un groupe anionique hydrosolubilisant carboxylique, sulfonate, sulfate, phosphate ou phosphonate et les alkylbétaïnes, les sulfobétaïnes, les amidobétaïnes ou les amidosulfobétaïnes.

14. Composition selon la revendication 13, caractérisée par le fait que les agents tensio-actifs amphotères sont choisis parmi :

- les amphocarboxyglycinates répondant à la formule : 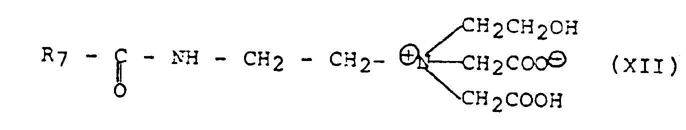 dans laquelle R₇ désigne un radical alkyle dérivé du coprah, un radical heptyle, nonyle ou undécyle; et
- les amphocarboxypropionates : 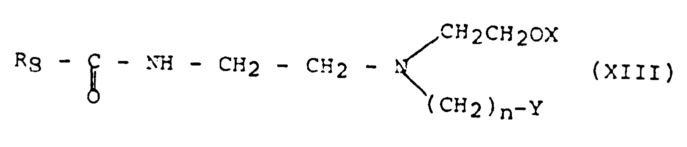 dans laquelle :
n est égal à 1 ou 2;
X désigne -CH₂CH₂COOH ou hydrogène;
Y désigne -COOH ou le radical 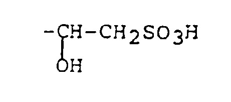
R₈ désigne le radical alkyle dérivé du coprah, un radical alkyle en C₇, Cg, C₁₁, C₁₃, un radical alkyle en C₁₇ et sa forme iso, un radical en C₁₇ insaturé, un radical alkyle dérivé de l'huile de lin.

15. Composition selon l'une quelconque des revendications 1 à 14, caractérisée par le fait que la composition contient un mélange d'agents tensio-actifs anioniques et d'agents tensio-actifs amphotères, zwittérioniques ou non-ioniques.

16. Composition selon la revendication 15, caractérisée par le fait que l'agent tensio-actif anionique est choisi parmi les alkyl(C₁₂-C₁₄)sulfates de sodium, de triéthanolamine ou d'ammonium, les alkyl (C₁₂-C₁₄)éthersulfates de sodium oxyéthylénés à 2,2 moles d'oxyde d'éthylène, le cocoyliséthionate de sodium et l'a-oléfine(C₁₄-C₁₆)Sulfonate de sodium, ainsi que leurs mélanges, et qu'il est utilisé avec

- soit un agent tensio-actif amphotère choisi parmi les amphocarboxyglycinates;
- soit les agents tensio-actifs zwittérioniques de la famille des alkylbétaïnes.

17. Composition selon l'une quelconque des revendications 1 à 16, caractérisée par le fait que les silicones sont présentes dans la composition dans des proportions comprises entre 0,2 et 20% en poids par rapport au poids total de la composition, et de préférence entre 0,2 et 10% en poids.

18. Composition selon l'une quelconque des revendications 1 à 17, caractérisée par le fait que les scléroglucanes sont utilisés dans des proportions comprises entre 0,1 et 5% en poids et en particulier entre 0,2 et 3% en poids.

19. Composition selon l'une quelconque des revendications 1 à 18, caractérisée par le fait que les agents tensio-actifs sont utilisés dans des proportions comprises entre 5 et 50% en poids par rapport au poids total de la composition, et de préférence entre 8 et 30% en poids.

20. Composition selon l'une quelconque des revendications 1 à 19, caractérisée par le fait que l'on utilise un mélange d'agents tensio-actifs anioniques et d'agents amphotéres ou zwittérioniques, dans lequel les agents tensio-actifs amphotères ou zwittérioniques représentent jusqu'à 50% en poids par rapport au poids total des agents tensio-actifs présents dans la composition.

21. Composition selon l'une quelconque des revendications 1 à 19, caractérisée par le fait que l'on utilise un mélange d'agents tensio-actifs non-ioniques et d'agents tensio-actifs anioniques, et que les agents tensio-actifs non-ioniques représentent jusqu'à 90% en poids par rapport à la quantité totale d'agents tensio-actifs présents dans la composition.

22. Composition selon l'une quelconque des revendications 1 à 21, caractérisée par le fait que le milieu aqueux est constitué uniquement par de l'eau ou un mélange eau/solvant cosmétiquement acceptable.

23. Composition selon l'une quelconque des revendications 1 à 22, caractérisée par le fait qu'elle a un pH compris entre 3 et 9, de préférence entre 4 et 8.

24. Composition selon l'une quelconque des revendications 1 à 23, caractérisée par le fait qu'elle contient en plus des agents régulateurs de viscosité choisis parmi les électrolytes, les hydrotropes, des épaississants autres que les scléroglucanes.

25. Composition selon l'une quelconque des revendications 1 à 24, caractérisée par le fait qu'elle contient en plus des agents tensio-actifs cationiques, des polymères anioniques, cationiques, amphotères ou des protéines éventuellement quaternisées.

26. Procédé de lavage et de conditionnement des cheveux, caractérisé par le fait que l'on applique sur des cheveux humides la composition telle que définie dans l'une quelconque des revendications 1 à 25 et qu'on procède ensuite au rinçage à l'eau.

27. Procédé de lavage de la peau, caractérisé par le fait que l'on applique sur celle-ci un gel douche ayant la composition telle que définie dans l'une quelconque des revendications 1 à 25 et qu'on procède ensuite à un rinçage à l'eau.

## Patentansprüche

1. Zusammensetzung zum Waschen keratinischer Materie, insbesondere der Haare und/oder der Haut, dadurch gekennzeichnet, daß sie in einem wässrigen Milieu mindestens enthält:

- ein anionisches, nicht ionisches oder amphoteres, waschwirksames oberflächenaktives Mittel;
- ein gegebenenfalls mit Glyoxal behandeltes Scleroglucan sowie
- ein im wässrigen Milieu unlösliches Silicon, ausgewählt aus:
A) flüchtigen Siliconen mit einem Siedepunkt von 60 bis 260°C;
B) nicht-flüchtigen Siliconen, ausgewählt aus:
a) Polyalkylsiloxanen, ausgewählt aus linearen Polydimethylsiloxanen mit Trimethylsilyl-Endgruppen mit einer Viskosität von 5 x10⁻⁶bis2,5m2/sbei 25°C, linearen Polydimethylsiloxanen mit Trihydroxysilyl-Endgruppen und aus Poly-C₁₋₂₀-alkylsiloxanen;
b) Polyaklylarylsiloxanen, ausgewählt aus linearen und/oderverzweigten Polymethylphenylsiloxanen oder Polydimethyldiphenylsiloxanen mit einer Viskosität von 10-⁵ bis 5 x 10-² m²/s bei 25°C;
c) modifizierten oder nicht-modifizierten Copolymeren von Polyethersiloxanen;
d) Silicon-Gummi aus Polydiorganosiloxanen mit erhöhtem Molekulargewicht von 200.000 bis 1.000.000, alleine verwendet oder in Mischung mit einem Lösungsmittel, ausgewählt aus flüchtigen Siliconen, Polydimethylsiloxan-Ölen (PDMS), Polyphenylmethylsiloxan-Ölen (PPMS), Isoparaffinen, Methylenchlorid, Pentan, Dodecan, Tridecan, Tetradecan oder aus deren Mischungen;
e) Organopolysiloxan-Harzen, die vernetzte Siloxan-Systeme sind, umfassend R₂Si0_{2/2}, RSi0_{3/2} und Si0_{4/2}-Einheiten, in denen Reine Kohlenwasserstoffgruppe mit 1 bis 6 Kohlenstoffatomen oder eine Phenylgruppe darstellt;
f) oben (untera) bis d)) definierten organomodifizierten Siliconen, enthaltend in deren allgemeiner Struktur eine oder mehrere organofunktionelle Gruppen, die an die Siloxankette direkt oder über das Zwischenglied eines Kohlenwasserstoffrestes gebunden und ausgewählt sind aus:
(i) Thiolgruppen,
(ii) Carboxylatgruppen;
(iii) Alkoxylgruppen;
(iv) Hydroxylgruppen;
(v) Acyloxyalkylgruppen;
(vi) anionischen Gruppen des Alkylcarboxyl-, 2-Hydroxyalkylsulfonat oder 2-Hydroxyalkylthiosulfat-Typs, wobei diese Zusammensetzung dann kein Polysiloxan mit β-Substitutionsfunktion enthält.

2. Zusammensetzung gemäß Anspruch 1,
dadurch gekennzeichnet, daß
das flüchtige Silicon aus zyklischen Siliconen mit 3 bis 7 Siliziumatomen und vorzugsweise mit 4 bis 5 Siliziumatomen, Dimethylsiloxan/Methylalkylsiloxan-Cyclopolymeren, gemäß der Formel:

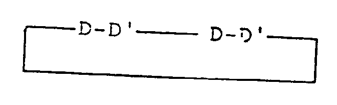

mit 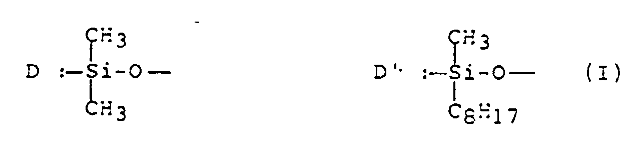 und aus linearen flüchtigen Siliconen ausgewählt ist, die 2 bis 9 Siliziumatome und eine Viskosität unterhalb oder gleich 5 x 10-^{s} m²/s bei 25°C aufweisen.

3. Zusammensetzung gemäß Anspruch 2, dadurch gekennzeichnet, daß die flüchtigen Silicone aus Octamethylcyclotetrasiloxan, Decamethylcyclopentasiloxan ausgewählt sind.

4. Zusammensetzung gemäß Anspruch 1, dadurch gekennzeichnet, daß der Silicon-Gummi aus Gummiprodukten von Poly(dimethylsiloxan/methylvinylsiloxan), Poly(dimethylsi- loxan/diphenylsiloxan), Poly(dimethylsiloxan/phenylmethylsiloxan), Poly(dimethylsiloxan/diphenylsilo- xan/methylvinylsiloxan) ausgewählt ist.

5. Zusammensetzung gemäß Anspruch 1, dadurch gekennzeichnet, daß der Silicon-Gummi in Mischung mit einem Lösungsmittel aus Mischungen aus einem am Kettenende hydroxylierten Polydimethylsiloxan und einem zyklischen Polydimethylsiloxan, einer Mischung aus einem Polydimethylsiloxan-Gummi mit einem zyklischen Silicon, einer Mischung aus einem Polydimethylsiloxan-Gummi und einem PDMS-ÖI unterschiedlicher Viskositäten ausgewählt ist.

6. Zusammensetzung gemäß Anspruch 1, dadurch gekennzeichnet, daß die Polyorganosiloxane mit Hydroxylgruppe Polyorganosiloxane mit Hydroxyalkylfunktion der Formel sind:

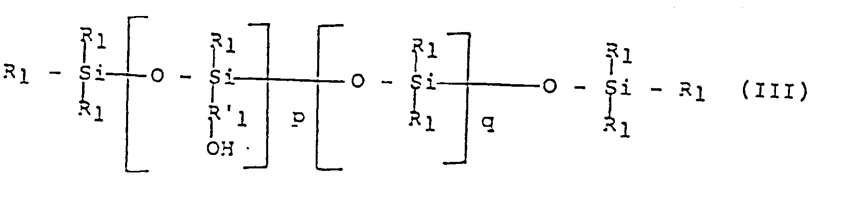

worin:
die Reste R_{1'} gleich oder verschieden, aus Methyl- und Phenylresten ausgewählt sind, wobei mindestens 60 Mol% der Reste R₁ eine Methylgruppe darstellen;
der Rest R'ᵢ eine zweiwertige C₂-₁₈-Alkylenkette ist;
p 1 bis 30 beträgt;
q 1 bis 150 beträgt.

7. Zusammensetzung gemäß jedem Anspruch 1 bis 6, dadurch gekennzeichnet, daß die Scleroglucane neutrale Polysaccharide mikrobiellen Ursprungs sind, erhältlich durch aerobe Fermentation in einem glucosierten Milieu mit einem Champignon des Typs Sclerotium, und die Struktur eines Homopolymers von D-Glucopyranose aufweisen.

8. Zusammensetzung gemäß Anspruch 7, dadurch gekennzeichnet, daß die anionischen oberflächenaktiven Mittel aus Alkali-, Ammonium-, Amin- oderAminoalkohol-Salzen der folgenden Verbindungen ausgewählt sind, und zwar sind diese Verbindungen:

- Alkylsulfate, Alkylethersulfate, Alkylamidethersulfate, Alkanolamidsulfate, Alkylarylpolyethersulfate, Monoglyceridsulfate;
- Alkylsulfonate, Alkylarylsulfonate, Alkylamidsulfonate, Olefinsulfonate, Paraffinsulfonate;
- Alkylsulfosuccinate, Alkylethersulfosuccinate, Alkylamidsulfosuccinate;
- Alkylsulfosuccinamate;
- Alkylsulfoacetate;
- Alkylphosphate; Alkyletherphosphate;
- Acylsarcosinate, Acylpolypeptidate, Acylisethionate, N-Acyltaurate, worin der Alkyl- oder Acylrest eine lineare Kohlenwasserstoffkette mit 12 bis 18 Kohlenstoffatomen ist.

9. Zusammensetzung gemäß jedem Anspruch 1 bis 8, dadurch gekennzeichnet, daß die anionischen oberflächenaktiven Mittel aus Salzen von Öl-, Ricinolen-, Palmitin-, Stearinsäuren, von Säuren des Öls von Kopra oder des hydrierten Öls von Kopra, Acyllactylaten, in denen der Acylrest 8 bis 20 Kohlenstoffatome enthält, ausgewählt sind.

10. Zusammensetzung gemäß jedem Anspruch 1 bis 7, dadurch gekennzeichnet, daß die nicht-ionischen oberflächenaktiven Mittel ausgewählt sind aus an der linearen Fettkette mit 8 bis 18 Kohlenstoffatomen polyoxethylierten, polypropoxylierten oder polyglycerierten Alkoholen, Alkylphenolen und Fettsäuren, aus Copolymeren von Ethylen- und Propylenoxid, Kondensaten von Ethylen- und Propylenoxiden mit Fettalkoholen, polyethoxylierten Fettamiden, polyethoxylierten Fettaminen, Ethanolamiden, Glycolfettsäureestern, oxethylierten oder nicht oxethylierten Sorbitanfettsäureestern, Saccharosefettsäureestern, Polyethylenglycolfettsäureestern, Phosphorsäuretriestern, Fettsäureestern von Glucosederivaten, Aminoxiden.

11. Zusammensetzung gemäß jedem Anspruch 1 bis 7, dadurch gekennzeichnet, daß die nicht-ionischen oberflächenaktiven Mittel aus Verbindungen der Formeln ausgewählt oder gemäß der folgenden Verfahren hergestellt sind:
a) 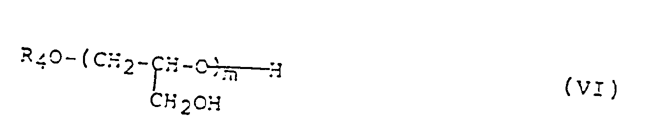 worin R₄ einen Alkylrest oder eine Mischung von Alkylresten mit 10 bis 14 Kohlenstoffatomen darstellt und m eine ganze Zahl oder Dezimalzahl von 2 bis 10 und vorzugsweise 3 bis 6 ist;
b) 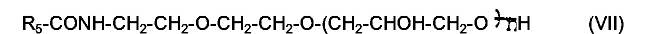 worin R₅ einen Alkyl- und/oder Alkenylrest oder eine Mischung dieser Reste mit 11 bis 17 Kohlenstoffatomen darstellt und n eine ganze Zahl oder Dezimalzahl vn 1 bis 5 und vorzugsweise von 1,5 bis 4 ist;
c) 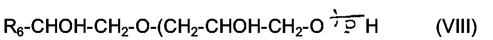 worin R₆ einen aliphatischen, cycloaliphatischen, arylaliphatischen Rest mit vorzugsweise 7 bis 21 Kohlenstoffatomen sowie einen Rest aus deren Mischungen darstellt, wobei die aliphatischen Ketten Alkylketten bedeuten, die 1 bis 6 Ether-, Thioether und/oder Hydroxymethylengruppen enthalten können, und worin p 1 bis 10 beträgt;
d) Verbindungen, die durch Konensation unter saurer Katalyse von 2 bis 10 und vorzugsweise 2,5 bis 6 Mol Glycidol pro Mol Alkohol oder a-Diol mit 10 bis 14 Kohlenstoffatomen hergestellt werden;
e) Verbindungen von Poly(hydroxypropylether), die durch Polyaddition von Glycerinmonochlorhydrin an eine polyhydroxylierte organische Verbindung in Gegenwart einer starken Base unter Entfernung des entstehenden Wassers durch Destillation hergestelltwerden.

12. Zusammensetzung gemäß Anspruch 11, dadurch gekennzeichnet, daß die nicht-ionischen oberflächenaktiven Mittel aus Verbindungen der Formeln ausgewählt sind:

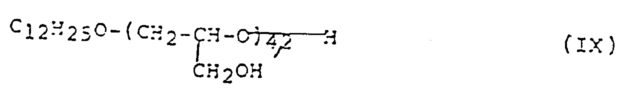

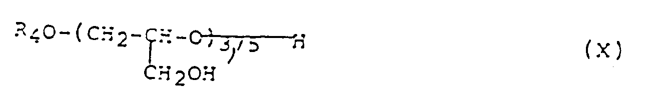

worin R₄ eine Mischung aus C₁₀H₂₁- und C₁₂H₂₅-Alkylresten bedeutet;
- aus Verbindungen, hergestellt durch Kondensation unter alkalischer Katalyse von 3,5 Mol Glycidol mit einem a-Diol mit 12 Kohlenstoffatomen;
- aus Verbindungen der Formel: 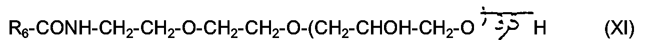 worin R₆ eine Mischung aus Resten darstellt, die die folgenden Alkyl- und Alkenylreste enthalten: C₁₁ H₂₃, C₁₃H₂₇, von Fettsäuren von Kopra abgeleitete Reste und den von Ölsäure abgeleiteten Rest;
- aus Verbindungen, hergestellt durch Kondensation von 3,5 Mol Glycidol mit einer Mischung von a-C₁₁-₁₄-Diolen;
- aus Poly(hydroxypropylether), erhältlich durch Kondensation von Glycerinmonochlorhydrin (2,5 Mol) in Gegenwart von Soda mit Dodecandiol-1,2.

13. Zusammensetzung gemäß jedem Anspruch 1 bis 7, dadurch gekennzeichnet, daß die amphoteren und zwitterionischen oberflächenaktiven Mittel aus sekundären oder tertiären aliphatischen Aminderivaten, in denen der aliphatische Rest eine lineare oder verzweigte Kette mit 8 bis 18 Kohlenstoffatomen ist und mindestens eine wasserlösliche anionische Carboxyl-, Sulfonat-, Sulfat-, Phosphat- oder Phosphonat-Gruppe enthält, sowie aus Alkylbetainen, Sulfobetainen, Amidobetainen oder Amidosulfobetainen ausgewählt sind.

14. Zusammensetzung gemäß Anspruch 13, dadurch gekennzeichnet, daß die amphoteren oberflächenaktiven Mittel ausgewählt sind aus:

- den Amphocarboxyglycinaten der Formel: 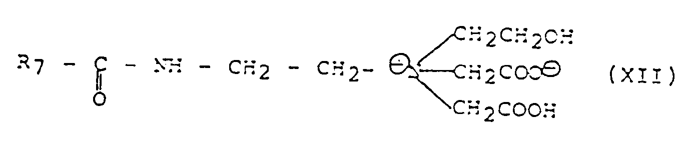 worin R₇ einen von Kopra abgeleiteten Alkyl-, einen Heptyl-, Nonyl- oder Undecylrest darstellt;
- den Amphocarboxypropionaten: 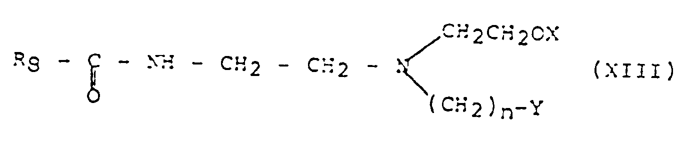 worin:
n 1 oder 2 bedeutet;
X die Gruppe -CH₂CH₂COOH oder ein Wasserstoffatom bedeutet;
Y -COOH oder den Rest 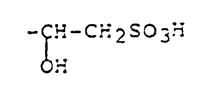 bedeutet;
R₈ einen von Kopra abgeleiteten Alkylrest, einen C₇-, Cg-, C₁₁-, C₁₃-Alkylrest, einen C₁₇-Alkylrest und seine Iso-Form, einen ungesättigten C₁₇-Rest, einen von Leinöl abgeleiteten Alkylrest bedeutet.

15. Zusammensetzung gemäß jedem Anspruch 1 bis 14, dadurch gekennzeichnet, daß die Zusammensetzung eine Mischung aus anionischen und aus amphoteren, zwitterionichen oder nicht-ionischen oberflächenaktiven Mitteln enthält.

16. Zusammensetzung gemäß Anspruch 15, dadurch gekennzeichnet, daß das anionische oberflächenaktive Mittel aus Natrium-, Triethanolamin- oder Ammonium-C₁₂-₁₄-alkylsulfaten, mit 2,2 Mol Ethylenoxid oxethylierten Natrium-C₁₂₋₁₄-alkylethersulfaten, Natriumcocoylisethionat und Natrium-C₁₄-₁₆-α-olefinsulfonatsowie aus deren Mischungen ausgewählt ist und verwendet wird mit

- entweder einem aus Amphocarboxyglycinaten ausgewählten amphoteren oberflächenaktiven Mittel;
- oder zwitterionischen oberflächenaktiven Mitteln der Familie der Alkylbetaine.

17. Zusammensetzung gemäß jedem Anspruch 1 bis 16, dadurch gekennzeichnet, daß die Silicone in der Zusammensetzung in Mengenverhältnissen von 0,2 bis 20 Gew.%, vorzugsweise von 0,2 bis 10 Gew.%, bezogen auf Gesamtgewicht der Zusammensetzung, vorliegen.

18. Zusammensetzung gemäß jedem Anspruch 1 bis 17, dadurch gekennzeichnet, daß die Scleroglucane in Mengenverhältnissen von 0,1 bis 5 Gew.% und insbesondere von 0,2 bis 3 Gew.% verwendet worden.

19. Zusammensetzung gemäß jedem Anspruch 1 bis 18, dadurch gekennzeichnet, daß

die oberflächenaktiven Mittel in Mengenverhältnissen von 5 bis 50 und vorzugsweise von 8 bis 30 Gew.%, bezogen auf Gesamtgewicht der Zusammensetzung, verwendet werden.

20. Zusammensetzung gemäß jedem Anspruch 1 bis 19, dadurch gekennzeichnet, daß man eine Mischung aus anionischen und amphoteren oder zwitterionischen oberflächenaktiven Mitteln verwendet, worin die amphoteren oder zwitterionischen oberflächenaktiven Mittel bis zu 50 Gew.% ausmachen, bezogen auf Gesamtgewicht der in der Zusammensetzung vorhandenen oberflächenaktiven Mittel.

21. Zusammensetzung gemäß jedem Anspruch 1 bis 19, dadurch gekennzeichnet, daß man eine Mischung aus nicht-ionischen und anionischen oberflächenaktiven Mitteln verwendet und die nicht-ionischen oberflächenaktiven Mittel bis zu 90 Gew.% ausmachen, bezogen auf die Gesamtmenge der in der Mischung vorhandenen oberflächenaktiven Mittel.

22. Zusammensetzung gemäß jedem Anspruch 1 bis 21, dadurch gekennzeichnet, daß das wässrige Milieu alleine aus Wasser oder aus einer Mischung aus Wasser und einem kosmetisch verträglichen Lösungsmittel zusammengesetzt ist.

23. Zusammensetzung gemäß jedem Anspruch 1 bis 22, dadurch gekennzeichnet, daß sie einen pH von 3 bis 9 und vorzugsweise von 4 bis 8 aufweist.

24. Zusammensetzung gemäß jedem Anspruch 1 bis 23, dadurch gekennzeichnet, daß sie zusätzlich Viskositätsregulierungsmittel enthält, ausgewählt aus Elektrolyten, Hydrotropen, anderen Verdickungsmitteln als den Scleroglucanen.

25. Zusammensetzung gemäß jedem Anspuch 1 bis 24, dadurch gekennzeichnet, daß sie zusätzlich kationische oberflächenaktive Mittel, anionische, kationische, amphotere Polymere oder gegebenenfalls quaternierte Proteine enthält.

26. Verfahren zum Waschen und Konditionieren der Haare, dadurch gekennzeichnet, daß man auf feuchte Haare die gemäß jedem der Ansprüche 1 bis 25 definierte Zusammensetzung aufbringt und im Anschluß daran eine Spülung mit Wasser durchführt.

27. Verfahren zum Waschen der Haut, dadurch gekennzeichnet, daß man auf sie ein Duschgel aufträgt, das die in jedem der Ansprüche 1 bis 25 definierte Zusammensetzung aufweist, und im Anschluß daran eine Spülung mit Wasser durchführt.

## Claims

1. Composition for washing keratinous materials, in particular hair and/or the skin, characterized in that it contains, in an aqueous medium, at least:
- one anionic, nonionic or amphoteric detergent surfactant;
- one scleroglucan optionally treated with glyoxal; and
- one silicone insoluble in the aqueous medium, chosen from:
A) volatile silicones having a boiling point between 60 and 260°C; and
B) non-volatile silicones, chosen from:
a) polyalkylsiloxanes chosen from straight-chain polydimethylsiloxanes containing trimethylsilyl end groups and having a viscosity of 5 x 10-⁶ to 2.5 m²/s at 25°C, and straight-chain polydimethylsiloxanes containing trihydroxysilyl end groups and poly(C₁-C₂₀)alkylsiloxanes;
b) polyalkylarylsiloxanes chosen from straight-chain and/or branched polymethylphenylsiloxanes or polydimethyldiphenylsiloxanes having a viscosity of 10-⁵ to 5 x 10-² m²/s at 25°C;
c) unmodified or modified polyether-siloxanes copolymers;
d) silicone gums consisting of polydiorgano- siloxanes having a high molecular weight of between 200,000 and 1,000,000, used on theirown or as a mixture in a solvent chosen from volatile silicones, polydimethylsiloxane (PDMS) oils, polyphenylmethylsiloxane (PPMS) oils, isoparaffins, methylene chloride, pentane, dodecane, tridecane and tetradecane or their mixtures;
e) organopolysiloxane resins which are cross- linked siloxane systems containing R₂SiO_{2/2}, RSiO_{3/2} and SiO_{4/2} units, in which units R represents a hydrocarbon group having 1 to 6 carbon atoms or a phenyl group;
f) the organo-modified silicones defined above (a to d) containing, on their general structure, one or more organo-functional groups fixed directly to the siloxane chain orfixed via a hydrocarbon radical and chosen from:
(i) thiol groups;
(ii) carboxylate groups;
(iii) alkoxy groups;
(iv) hydroxyl groups;
(v) acyloxyalkyl groups; and
(vi) anionic groups of the alkylcarboxylic 2-hydroxyalkylsulphonate or 2-hydroxy- alkylthiosul- phate type this composition not containing polysiloxane with a β-ketoester functional group.

2. Composition according to Claim 1, characterized in that the volatile silicone is chosen from the cyclic silicones containing 3 to 7 silicon atoms and preferably 4 to 5 silicon atoms, the dimethylsiloxane/me- thylalkyl- siloxane copolymers corresponding to the formula: 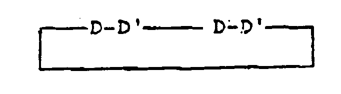 where 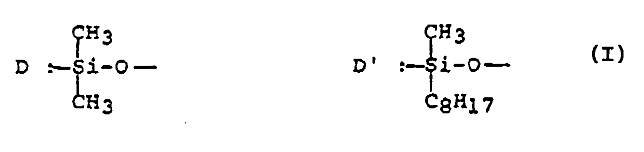 and the straight-chain volatile silicones having 2 to 9 silicon atoms and a viscosity of less than or equal to 5 x 10-^{s} m²/s at 25°C.

3. Composition according to Claim 2, characterized in that the volatile silicones are chosen from octamethylcyclotetrasiloxane and decamethylcyclopenta- siloxane.

4. Composition according to Claim 1, characterized in that the silicone gums are chosen from the poly(dimethylsiloxane/methylvinylsiloxane), poly(dimethylsiloxane/diphenylsiloxane), poly(dimethyl- siloxane/phenylmethylsiloxane) and poly(dimethylsiloxane/diphenylsiloxane/methylvinylsiloxane) gums.

5. Composition according to Claim 1, characterized in that the silicone gums as a mixture in a solvent are chosen from the mixtures of a polydimethylsiloxane hydroxylated at the end of the chain and a cyclic polydimethylsiloxane; a mixture of a polydimethylsiloxane gum with a cyclic silicone; and a mixture of a polydimethyl- siloxane gum and a PDMS oil of different viscosities.

6. Composition according to Claim 1, characterized in that the polyorganosiloxanes containing a hydroxyl group are polyorganosiloxanes containing a hydroxyalkyl function, corresponding to the formula:

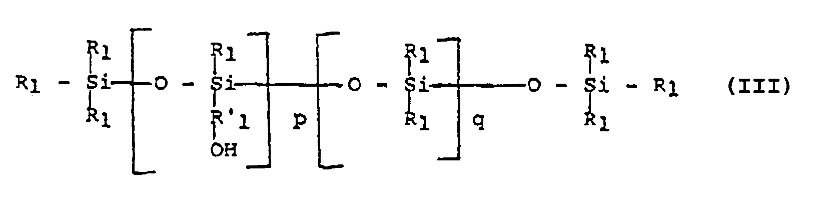

in which:
the radicals Rᵢ, which are identical or different, are chosen from methyl and phenyl radicals, at least 60 mole% of the radicals R₁ denoting methyl;
the radical R'ᵢ is a C₂-C₁₈ divalent hydrocarbon alkylene member;
p is between 1 and 30 inclusive; and
q is between 1 and 150 inclusive.

7. Composition according to any one of Claims 1 to 6, characterized in that the scleroglucans are neutral polysaccharides of microbial origin, obtained by aerobic fermentation of a glucose medium by a mushroom of the Sclerotium type and having the structure of a homopolymer of D-glucopyranose.

8. Composition according to Claim 7, characterized in that the anionic surfactants are chosen from the alkali metal salts, the ammonium salts, the amine salts or the amino alcohol salts of the following compounds:

- alkyl sulphates, alkyl ether-sulphates, alkylamide ether-sulphates, alkanolamide sulphates, alkylar- ylpolyether sulphates and monoglyceride sulphates;
- alklylsulphonates, alkylarylsulphonates, alkylamidesulphonates, olefinsulphonates and paraffinsul- phonates;
- alkyl sulphosuccinates, alkyl ether-sulphosuccinates and alkylamide sulphosuccinates;
- alkyl sulphosuccinamates;
- alkyl sulphoacetates;
- alkyl phosphates and alkyl ether-phosphates; and
- acyl sarcosinates, acyl polypeptidates, acyl isethionates and N-acyltaurates, in which the alkyl or acyl radical is a straight hydrocarbon chain of 12 to 18 carbon atoms.

9. Composition according to any one of Claims 1 to 8, characterized in that the anionic surfactants are chosen from the salts of oleic, ricinoleic, palmitic and stearic acids; the acids of copra oil or hydrogenated copra oil; and acyl lactylates in which the acyl radical contains 8 to 20 carbon atoms.

10. Composition according to any one of Claims 1 to 7, characterized in that the nonionic surfactants are chosen from the polyoxyethylenated, polypropoxylated or polyglycerolated alcohols, alkylphenols and fatty acids having a straight fatty chain containing 8 to 18 carbon atoms; the copolymers of ethylene oxide and propylene oxide, the condensation products of ethylene oxide and propylene oxide with fatty alcohols, polyethoxylated fatty amides, polyethoxylated fatty amines, ethanolamides, glycol fatty acid esters, sorbitan fatty acid esters, which may or may not be oxyethylenated, sucrose fatty acid esters, polyethylene glycol fatty acid esters, phosphoric triesters, fatty acid esters of glucose derivatives and amine oxides.

11. Composition according to any one of Claims 1 to 7, characterized in that the nonionic surfactants are chosen from the compounds corresponding to the following formulae or prepared by the following processes:
a) 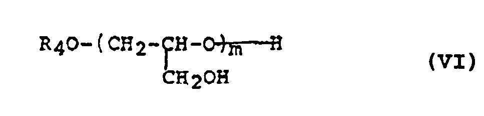 in which R₄ denotes an alkyl radical containing 10 to 14 carbon atoms or a mixture of such radicals and m is an integer or decimal number from 2 to 10 and preferably from 3 to 6;
b) 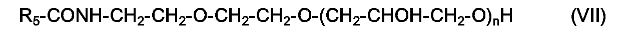 in which R₅ denotes an alkyl and/or alkenyl radical having from 11 to 17 carbon atoms, or a mixture of such radicals, and n denotes an integer or decimal number from 1 to 5 and preferably from 1.5 to 4;
c) 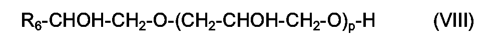 in which R₆ denotes an aliphatic, cycloaliphatic or arylaliphatic radical, preferably having 7 to 21 carbon atoms, and their mixtures, the aliphatic chains denoting alkyl chains which can contain 1 to 6 ether, thioether and/or hydroxymethylene groups, and p is between 1 and 10 inclusive;
d) the compounds prepared by a condensation reaction, under acid catalysis, of 2 to 10 and preferably 2.5 to 6 moles of glycidol per mole of alcohol or alpha- diol containing 10 to 14 carbon atoms;
e) the poly(hydroxypropyl ether) compounds prepared by polyaddition of glycerol monochlorohydrin to an organic polyhydroxy compound, in the presence of a strong base, with removal of the water by distillation at the rate at which it is formed.

12. Composition according to Claim 11, characterized in that the nonionic surfactants are chosen from the compounds corresponding to the formulae: 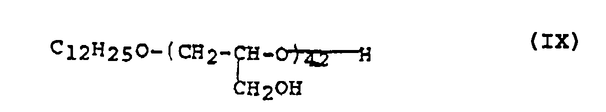 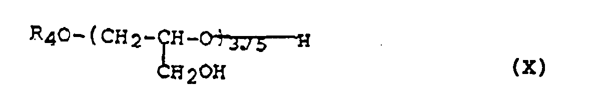 in which R₄ denotes a mixture of C_{1O}H₂₁ and C₁₂H₂₅ alkyl radicals;
- the compounds prepared by a condensation reaction, under alkaline catalysis, of 3.5 moles of glycidol with an alpha-diol having 12 carbon atoms;
- the compounds corresponding to the formula: 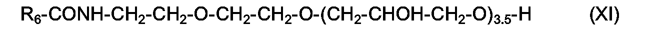 in which R₆ denotes a mixture of radicals comprising the following alkyl and alkenyl radicals: C₁₁H₂₃, C₁₃H₂₇, the radicals derived from copra fatty acids and the radical derived from oleic acid;
- the compounds prepared by a condensation reaction of 3.5 moles of glycidol with a mixture of C₁₁-Cᵢ₄ alpha-diols;
- the poly(hydroxypropyl ether) obtained by a condensation reaction of glycerol monochlorohydrin (2.5 moles), in the presence of sodium hydroxide, with dodecane-1,2-diol.

13. Composition according to any one of Claims 1 to 7, characterized in that the amphoteric and zwitterionic surfactants are chosen from the derivatives of secondary or tertiary aliphatic amines in which the aliphatic radical is a straight or branched chain containing from 8 to 18 carbon atoms and contains at least one anionic carboxyl, sulphonate, sulphate, phosphate or phosphonate group conferring solubility in water, and the alkylbetaines, sulphobetaines, amidobetaines or amidosulphobetaines.

14. Composition according to Claim 13, characterized in that the amphoteric surfactants are chosen from:
- the amphocarboxyglycinates corresponding to the formula: 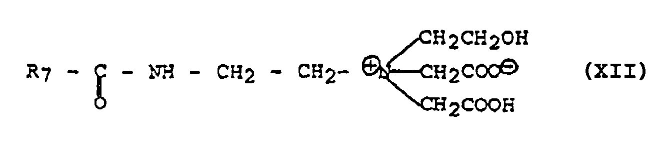 in which R₇ denotes an alkyl radical derived from copra, or a heptyl, nonyl or undecyl radical; and
- amphocarboxypropionates 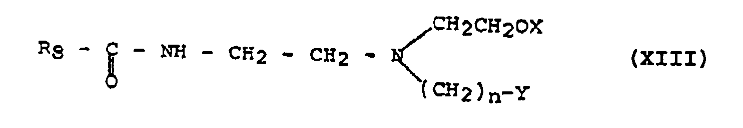 in which:
n is equal to 1 or 2;
X denotes -CH₂CH₂COOH or hydrogen;
Y denotes -COOH or the radical 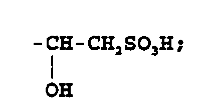 and
R₈ denotes the alkyl radical derived from copra, a C₇, C₉, C₁₁ or C₁₃ alkyl radical, a C₁₇ alkyl radical and its iso form, an unsaturated C₁₇ radical or an alkyl radical derived from linseed oil.

15. Composition according to any one of Claims 1 to 14, characterized in that the composition contains a mixture of anionic surfactants and amphoteric, zwitterionic or nonionic surfactants.

16. Composition according to Claim 15, characterized in that the anionic surfactant is chosen from sodium (C₁₂-C₁₄)alkyl sulphates, triethanolamine (C₁₂-C₁₄)alkyl sulphates or ammonium (C₁₂-C₁₄)alkyl sulphates, oxyethylenated sodium (C₁₂-C₁₄)alkyl ether-sulphates containing 2.2 moles of ethylene oxide, sodium co- coylisethionate and sodium (C₁₄-C₁₆)-α-olefinsulphonate, as well as their mixtures, and in that it is used with

- either an amphoteric surfactant chosen from the amphocarboxyglycinates;
- or the zwitterionic surfactants of the family of the alkylbetaines.

17. Composition according to any one of Claims 1 to 16, characterized in that the silicones are present in the composition in proportions of between 0.2 and 20% by weight relative to the total weight of the composition, and preferably between 0.2 and 10% by weight.

18. Composition according to any one of Claims 1 to 17, characterized in that the scierogiucans are used in proportions of between 0.1 and 5% by weight and in particular between 0.2 and 3% by weight.

19. Composition according to any one of Claims 1 to 18, characterized in that the surfactants are used in proportions of between 5 and 50% by weight relative to the total weight of the composition, and preferably between 8 and 30% by weight.

20. Composition according to any one of Claims 1 to 19, characterized in that a mixture of anionic surfactants and amphoteric or zwitterionic agents is used in which the amphoteric or zwitterionic surfactants represent up to 50% by weight relative to the total weight of the surfactants present in the composition.

21. Composition according to any one of Claims 1 to 19, characterized in that a mixture of nonionic surfactants and anionic surfactants is used and in that the nonionic surfactants represent up to 90% by weight relative to the total amount of surfactants present in the composition.

22. Composition according to any one of Claims 1 to 21, characterized in that the aqueous medium consists solely of water or a cosmetically acceptable water/solvent mixture.

23. Composition according to any one of Claims 1 to 22, characterized in that it has a pH of between 3 and 9, preferably between 4 and 8.

24. Composition according to any one of Claims 1 to 23, characterized in that it also contains viscosity regulators chosen from electrolytes, hydrotopic agents and thickeners other than the scleroglucans.

25. Composition according to any one of Claims 1 to 24, characterized in that it also contains cationic surfactants, anionic, cationic or amphoteric polymers or optionally quaternized proteins.

26. Procedure for washing and conditioning hair, characterized in that the composition as defined in any one of Claims 1 to 25 is applied to wet hair and in that rinsing with water is then carried out.

27. Procedure for washing the skin, characterized in that a shower gel having the composition as defined in any one of Claims 1 to 25 is applied to the skin and in that rinsing with water is then carried out.
